# EUROPEAN PATENT SPECIFICATION

(11) **EP 1 390 031 B1**
(45) Date of publication and mention of the grant of the patent: **09.02.2005**
(21) Application number: 02714197.7
(22) Date of filing: 28.03.2002
(51) Int. Cl.: A61K 31/415, A61K 31/325, A61K 31/495, A61K 31/535, A61K 31/44, A61K 31/405, A61K 31/54, A61P 31/10

(54) **TOPICAL COMPOSITION CONTAINING A FUNGICIDE**
FUNGIZIDE ENTHALTENDE TOPISCHE ZUSAMMENSETZUNG
COMPOSITION TOPIQUE CONTENANT UN FONGICIDE

(30) Priority: 30.03.2001 GB 0108082
(43) Date of publication of application: 25.02.2004
(73) Proprietor: Novartis Consumer Health S.A., 1260 Nyon (CH)
(72) Inventor: LARNIER, Catherine, CH-1297 Founex (CH); STEIGER, Michel, CH-1814 La Tour-de-Peilz (CH)
(74) Representative: Grubb, Philip William
(86) International application number: PCT/EP2002/003547
(87) International publication number: WO 2002/078648

(56) References cited:
- EP-A- 0 510 561
- WO-A-00/28821
- L.DRAGO E.A.: "Antimycotic activity and phagocytosis effects of econazole in combination with ibuprofen isobuthanolium against vaginal strains" JOURNAL OF CHMOTHERAPY, vol. 12, no. 6, 2000, pages 509-515, XP008013678
- V.N.TARIO E.A.: "Use of decimal assay for additivity to demonstrate synergy in pair combinations of econazole, nikkomycin Z, and ibuprofen against candida albicans in vitro" ANTIMICROBIAL AGENTS AND CHEMOTHERAPY, vol. 39, no. 12, 1995, pages 2615-2619, XP002196359
- DATABASE BIOSIS [Online] BIOSCIENCES INFORMATION SERVICE, PHILADELPHIA, PA, US; 2000 P.LANZA ET AL: "Efficacy and safety of a 7-day course of miconazole associated with ibuprofen-isobutanolammonium compared to therapy with miconazole alone in mycotic vaginitis" Database accession no. PREV200000469532 XP002231002 & GAZZETTA MEDICA ITALIANA ARCHIVIO PER LE SCIENZE MEDICHE, vol. 159, no. 2, 2000, pages 71-78,

## Description

The invention relates to topical pharmaceutical compositions with antimycotic activity, more specifically anti-dermatophyte activity.

Dermatophytes are fungi that can cause infections of the skin, hair and nails due to their ability to utilize keratin. The organisms colonize the keratin tissues and cause fungal infections, e.g. known as tinea or ringworm, in association with the infected body part. The organisms are transmitted by either direct contact with infected host (human or animal) or by direct or indirect contact with infected exfoliated skin or hair in combs, hair brushes, clothing, furniture, theatre seats, caps, bed linens, towels, hotel rugs and locker room floors. Depending on the species the organism may be viable in the environment for up to 15 months. There is an increased susceptibility to infection when there is a pre-existing injury to the skin such as scares, bums, marching, excessive temperature and humidity.

The topical application of antifungal drugs, like terbinafine, in the treatment of fungal infections, such as mycoses, especially dermatomycoses caused by dermatophytes, e.g. athlete's foot (= tinea pedis), jock itch (= tinea cruris), ringworm, (e.g. facial) seborrheic dermatitis, or onychomycosis, is known in the art.

It has now surprisingly been found that by topical application of certain selected antifungals - in particular terbinafine - together with certain selected second drugs - in particular diclofenac and indomethacin - the antimycotic properties are improved in an unexpected manner. Surprisingly, the combinations of the present invention are particularly beneficial in fighting dermatophytes. As already outlined above, the latter are the main cause for superficial mycoses frequently occurring in humans, such athlete's foot, jock itch or ringworm. Treatment of said superficial mycoses is generally improved by use of the specific combinations of the invention. This is quite surprising in view of the fact that the antifungals concerned are known to be rather effective in the eradication and treatment of dermatophytes even when applied alone.

There are great differences between Candida infections, e.g. with Candida albicans, and those caused by dermatophytes: Candida infections are in general much more difficult to treat with antifungals and are often systemic. Dermatophytes, in contrast to Candida, never become pathogenic systemically. Candida species, in contrast to dermatophytes, are yeasts, are normally present in humans and usually become pathogenic only in case of overgrowth, often induced by local factors like immunodepression. The physiopathology of Candida and dermatophyte infections is completely different: Yeasts like Candida are opportunistic agents and usually need co-factors to become pathogenic, predominantly systemically. Dermatophytes, however, become immediately pathogenic when present, and on the skin exclusively.

With the combinations of the present invention, the cure of superficial mycoses caused by dermatophytes, e.g. athlete's foot, is in general achieved more quickly and a quicker relief of typical symptoms, such as itching, erythema, vesiculation, burning or fissures, is observed.

Therefore, the invention relates to a pharmaceutical composition adapted to topical administration comprising an antifungal selected from terbinafine and topically acceptable salts there of, and a second drug selected from the group consisting of diclofenac and indomethacin, and topically acceptable salts of any of said compounds, together with at least one topically acceptable carrier.

All the antifungals and drugs concerned are known and e.g. described in The Merck Index, Twelfth Edition, 1996, for example:

Terbinafine can be found under No. 9299; it is commercially available under the trademark LAMISIL. Topically acceptable salts thereof are e.g. terbinafine hydrochloride, terbinafine lactate or terbinafine ascorbate. Preferred are terbinafine and terbinafine hydrochloride, in particular terbinafine (= free base).

Diclofenac (free acid) can be found under No. 3132; it is commercially available under the trademark VOLTAREN. Topically acceptable salts thereof are e.g. diclofenac sodium, diclofenac potassium, diclofenac diethylammonium and diclofenac epolamine. Preferred is diclofenac sodium.

Indomethacin (free acid) can be found under No.4998. Topically acceptable salts thereof are e.g. indomethacin sodium (e.g. the trihydrate) or the megiumine salt of indomethacin (megiumine = N-methyl-D-glucamine). Preferred is indomethacin sodium.

Preferably, the invention relates to topical compositions, wherein the antifungal is selected from terbinafine and topically acceptable salts there of and the second drug is selected from the group consisting of diclofenac and indomethacin and topically acceptable salts of any of said compounds - as well as to the use thereof.

Especially, the invention relates to topical compositions, wherein the antifungal is selected from the group consisting of terbinafine and topically acceptable salts thereof, and the second drug is selected from the group consisting of diclofenac, indomethacin, and topically acceptable salts of any of said compounds.

In particular, the invention relates to topical compositions, wherein the antifungal is terbinafine, or a topically acceptable salt thereof, and the second drug is diclofenac, or a topically acceptable salt thereof.

In particular preferred is the combination of terbinafine (free base) and diclofenac sodium.

A further embodiment of the invention is characterized by topical compositions, wherein the antifungal is terbinafine, or a topically acceptable salt thereof, and the second drug is indomethacin, or a topically acceptable salt thereof. Another embodiment of the invention is characterized by topical compositions, wherein the antifungal is terbinafine, or a topically acceptable salt thereof, and the second drug is ibuprofen, or a topically acceptable salt thereof.

The topically acceptable carriers used largely depend on the kind of topical composition involved (see below). They include e.g. aqueous phases, oily phases or emulsions but on the other hand also e.g. bandage materials or a transdermal patch environment.

The topical compositions of the invention have valuable pharmacological properties. Especially, they are beneficial in the treatment of infections caused by dermatophytes, such as athlete's foot, jock itch, ringworm, or onychomycosis.

It has surprisingly been found that after administration of the topical compositions of the invention patients are relieved more quickly of the symptoms accompanying superficial mycoses, such as itching, erythema, vesiculation, burning or fissures, and said superficial mycoses are in general cured more quickly.

The beneficial properties of the topical compositions of the invention can be demonstrated, for example, in the following tests.
(1) Experimental dermatophytosis model in guinea pig: It can be shown that the course of infection is stopped very effectively by the topical compositions of the invention [see S. Fujita, Congress of the International Society for Human and Animal Mycology, Abstract S23 (1997)].
(2) Controlled double-blind comparative study, involving 600 patients with established tinea pedis who are randomized to three groups of 200 each undergoing either treatment with terbinafine/diclofenac sodium (1.0%/0.5%), terbinafine/indomethacin sodium (1%/0.5%), terbinafine alone (1.0%), diclofenac sodium alone (0.5%), indomethacin sodium alone (0.5%) or placebo (vehicle). Relief of symptoms after 1, 2 and 3 hours, 24 hours and then daily during the whole treatment period of 7 days is determined.
(3) Controlled double-blind comparative study, involving 600 patients with established tinea cruris who are randomized to three groups of 200 each undergoing either treatment with terbinafine/diclofenac sodium (1.0%/0.25%), terbinafine/indomethacin sodium (1.0%/0.25%), terbinafine alone (1.0%) or placebo (vehicle). Relief of symptoms after 1, 2 and 3 hours, 24 hours and then daily during the whole treatment period of 7 days is determined.
(4) Controlled double-blind comparative study, involving 570 patients with established tinea pedis who are randomized to three groups of 190 each undergoing either treatment with terbinafine/diclofenac sodium (1.0%/0.1%), terbinafine/indomethacin sodium (1.0%/0.1%), terbinafine alone (1.0%) or placebo (vehicle). Efficacy, i.e. clinical and mycological cure, is determined at 5 days, 7 days and week 6 after the beginning of treatment.

The .topical compositions of the invention are likewise beneficial in the treatment of animals, especially pets and farm animals, in an analogous manner as described herein for human treatment. Therefore the invention also relates to topical veterinary compositions which are composed in the same way as the topical pharmaceutical compositions described herein.

In the topical compositions of the invention, the antifungal component - in particular terbinafine - is typically present in an amount of from 0.1 up to 10%, especially of from 0.2 up to 5%, and in particular of from 0.5 up to 2%, of the total composition on a weight basis.

In the topical compositions of the invention, the second drug, e.g. diclofenac or indomethacin, is typically present in an amount of from 0.05 up to 10%, especially of from 0.1 up to 5%, and in particular of from 0.1 up to 2%, of the total composition on a weight basis. A particular embodiment of the invention is formed by those topical compositions, wherein the second drugn is present in an amount of from 0.1 up to 0.7%, especially of from 0.1 up to 0.5% and in particular of from 0.1 up to 0.3% of the total composition.

Preferably, the topically administered pharmaceutical compositions according to the invention comprise both the antifungal and the second drug in pharmacologically effective amounts.

The daily dosage of the active ingredients may depend on various factors, such as sex, age, weight and individual condition of the patient. The topical pharmaceutical compositions, e.g. in the form of emulsion-gels, creams or ointments, may be applied once, twice or three times daily. But also more frequent daily applications are possible. Patches and bandages may be applied, for example, once or twice daily.

The invention further relates to the use of an antifungal selected from of terbinafine, and topically acceptable salts of any of said compounds, and a second drug selected from the group consisting of diclofenac and indomethacin and topically acceptable salts of any of said compounds, (for the manufacture of a pharmaceutical composition adapted to topical administration) for the prevention or treatment of fungal infections, in particular dermatomycoses caused by dermatophytes.

Moreover, the invention relates to a method of treating fungal infections which comprises topically administering to a mammal in need thereof a therapeutically effective amount of a mixture of an antifungal selected from the group consisting of terbinafine and topically acceptable salts of any of said compounds, and a second drug selected from diclofenac, and indomethacin, and topically acceptable saits of any of said compounds.

Pharmaceutical compositions suitable for topical administration are e.g. creams, lotions, ointments, microemulsions, fatty ointments, gels, foam gels, emulsion-gels, nail lacquers (varnishes), shampoos, pastes, foams, tinctures, solutions, patches, bandages and transdermal therapeutic systems; preferred are emulsion-gels, gels, foam gels, creams, lotions, solutions, shampoos and nail lacquers. The manufacture and composition of such topical pharmaceutical compositions are known in the art (see e.g. WO 98/00168 A1, pages 8-15 or US patent 5,681,849).

In a particular embodiment of the invention, topical compositions are provided wherein the two active substances of the composition are essentially separated from each other by being dissolved in different phases so that interaction between both is minimized. What essentially is prevented by doing so is the formation of salts between the antifungal, e.g. terbinafine, and the second drug drug, e.g. diclofenac or indomethacin. Thereby typically the antifungal component, e.g. terbinafine, is dissolved or suspended in an oily phase, whereas the second drug, e.g. diclofenac or indomethacin, is dissolved or suspended in an aqueous phase.

The invention therefore further relates to a pharmaceutical composition adapted to topical administration in the form of an emulsion comprising an oily phase comprising an antifungal - as defined hereinbefore and hereinafter, in particular terbinafine -, or a topically acceptable salt thereof, and an aqueous phase comprising water, one or more solvents selected from the group consisting of C₁-C₄-alkanols, poly-hydroxy-C₂-C₅-alkanes and poly-C₂-C₅-alkylene glycols - especially a C₁-C₄-alkanol -, a water-soluble or water-miscible nonionic surfactant, wherein no anionic surfactant is present, and a second drug - as defined hereinbefore and hereinafter, especially diclofenac or indomethacin, and in particular diclofenac -, or a topically acceptable salt thereof.

The emulsions formed are e.g. emulsion gels or fluid emulsions, and they may comprise the active substances in dissolved or suspended form.

For the oily phase, any topically acceptable oil or lipid can be used (see e.g. the "fatty phase constituents" mentioned in US patent 4,917,886, columns 4-5). Preferred is isopropyl myristate or a mixture of coco-caprylate/caprate (= a mixture of caprylic/capric acid esters of C₁₂-C₁₈ fatty alcohols, e.g. Cetiol LC) and liquid paraffin. The oily phase is e.g. present in an amount of from 2-40%, preferably 2-30%, more preferably 2-15%, in particular 4-10%, (w/w) of the total composition. The weight ratio of the terbinafine component and the oily phase is typically of from 1:3 up to 1:40, preferably of from 1:4 up to 1:20.

The weight ratio of the antifungal component - in particular terbinafine - and the second drug component - especially diclofenac or indomethacin, and in particular diclofenac - is typically of from 1:0.05 up to 1:5, and preferably of from 1:0.1 up to 1:2.

Preferably the amount of water in a said emulsion is 50 to 85% (w/w) of the total composition. Preferably the amount of lower alkanol is 5 to 35% (w/w) of the total composition. A C₁-C₄-alkanol preferably is a physiologically acceptable C₁-C₄-alkanol, e.g. isopropanol or, preferably, ethanol. Poly-hydroxy-C₂-C₅-alkanes have at least two hydroxy groups, preferably 2, 3 or 4, and in particular 2 or 3 hydroxy groups. Preferred as C₂-C₅-alkanes are C₂-C₄-alkanes, and in particular ethane or propane. Preferred poly-hydroxy-C₂-C₅-alkanes are glycerin, ethylene glycol and propylene glycol. Poly-C₂-C₅-alkylene glycols are e.g. polyethylene glycol or polypropylene glycol, each typically having a molecular weight of from 200 up to 12000, preferably of from 250 up to 6000 and especially of from 300 up to 1500.

Examples of water-soluble or water-miscible nonionic surfactants are: (a) Reaction products of a natural or hydrogenated castor oil and ethylene oxide, e.g. the various tensides available under the tradename Cremophor, such as Cremophor RH 40, Cremophor RH 60 or Cremophor EL. Also suitable in this category are the various tensides available under the tradename Nikkol, e.g. Nikkol HCO-60. (b) Polyoxyethylene-sorbitan-fatty acid esters or polysorbates, e.g. of the type known and commercially available under the tradenames Tween and Armoran, such as Tween 20 [polyoxyethylene(20)sorbitanmonolaurate], Tween 40, 60, 65, 80, 85, 21, 61 or 81. (c) Polyoxyethylene fatty acid esters, e.g. polyoxyethylene stearic acid esters such as those known and commercially available under the tradename Myrj, or polyoxyethylene glycerin fatty acid esters, e.g. Cetiol HE (= PEG-7 glyceryl cocoate). (d) Polyoxyethylene-polyoxypropylene co-polymers e.g. of the type known and commercially available under the tradenames Pluronic and Emkalyx. (e) Polyoxyethylene fatty alcohol ethers, e.g. polyoxyethylene stearyl ether, oleyl ether, or cetyl ether, e.g. of the type known and commercially available under the tradenames Brij, e.g. Brij 78 or 96, and Cetomacrogol 1000. (f) Sorbitan-mono-fatty acid esters, e.g. sorbitan monolaurate (Span 20).

Conventional further excipients in said emulsions - as well as in the topical compositions of the invention in general - are, in particular, thickeners, such as carbomers (polyacrylic acid derivatives) as known and commercially available under the tradename Carbopol, e.g. Carbopol 974, 980 or 1342.

Said emulsions may be obtained e.g. by a process comprising dissolving the antifungal component - in particular terbinafine - and optionally further excipients as appropriate in the oil forming the oil phase. The latter may then be emulsified with the water phase (comprising water, one or more solvents selected from the group consisting of C₁₋C₄-alkanols, poly-hydroxy-C₂-C₅-alkanes and poly-C₂-C₅-alkylene glycols - especially a C₁-C₄-alkanol -, a nonionic surfactant, the second drug component, e.g. diclofenac or indomethacin, and optionally further excipients as appropriate). Optionally, the emulsions obtained are finally incorporated into a pre-prepared gel concentrate comprising the thickener and further excipients as appropriate. In that case, the thickener (carbomer) is preferably neutralized before being mixed with the emulsion.

Further conventional excipients in said emulsions - as well as in the topical compositions of the invention in general - are e.g. complexing agents, additives to adjust the pH, antimicrobial preservatives, antioxidants, flavours or colorants.

The following examples are intended to illustrate the invention.

### Example 1: A gel comprising 1% terbinafine hydrochloride and 1% diclofenac sodium is manufactured as follows.

| Ingredients | | Amount (g/100g) |
|---|---|---|
| (A) | terbinafine HCl | 1.00 |
| (B) | diclofenac sodium | 1.00 |
| (C) | sodium pyrosulfite | 0.02 |
| (D) | disodium edetate dihydrate (e.g. Komplexon III) | 0.02 |
| (E) | propylene glycol | 0.70 |
| (F) | hydroxypropyl cellulose (e.g. Klucel HF) | 2.00 |
| (G) | Polysorbate 20 (e.g. Tween 20) | 2.00 |
| (H) | ethanol 96% (v/v) | 35.00 |
| (I) | water, demineralized | ad 100.0 |
| (i) | Dissolve A in a mixture of E and H. | |
| (ii) | Dissolve B, C, D and G in I. | |
| (iii) | Mix (i) and (ii) at room temperature and add F. | |

### Example 2: An emulsion-gel comprising 1% terbinafine free base and 0.5% diclofenac sodium is manufactured as follows.

| Ingredients | | Amount (g/100g) |
|---|---|---|
| (A) | terbinafine free base | 1.00 |
| (B) | diclofenac sodium | 0.50 |
| (C) | Butyl hydroxy toluene | 0.02 |
| (D) | sodium hydroxide (pellets) | 0.10 |
| (E) | benzyl alcohol | 0.50 |
| (F) | Carbopol 974 P (carbomer) [= acrylic acid polymerisate] | 1.00 |
| (G) | sorbitan monolaurate (e.g. Span 20) | 1.00 |
| (H) | Polysorbate 20 (e.g. Tween 20) | 5.00 |
| (I) | ethanol 96% (v/v) | 10.00 |
| (J) | isopropyl myristate | 10.00 |
| (K) | water, demineralized | ad 100.0 |

(i) A, J, C, E, G and H are mixed together with slight warming until all solid particles are dissolved.
(ii) In an appropriate vessel or processor containing a stirrer and a homogenizer about half of K is heated to 60-70°C, and B is dissolved therein.
(iii) (i) is slowly added to (ii) while stirring and homogenizing until a homogeneous emulsion with appropriate droplet size is obtained. The concentrated emulsion is then cooled to room temperature.
(iv) In a separate vessel a basic carbomer gel is prepared by dispersing carbomer F in I and the second half of K and neutralizing with D.
(v) The basic emulsion (iii) is added to the basic gel and the whole is stirred at room temperature until a homogeneous emulsion gel is obtained.

### Example 3: An emulsion-gel comprising 1% terbinafine free base and 0.25% diclofenac sodium is manufactured as follows.

| Ingredients | | Amount (g/100g) |
|---|---|---|
| (A) | terbinafine free base | 1.0 |
| (B) | diclofenac sodium | 0.25 |
| (C) | isopropanol | 20.0 |
| (D) | polyethylene glycol 300 | 3.0 |
| (E) | polyhydroxyethylene cetyl stearyl ether (e.g. Cetomacrogol 1000) | 2.0 |
| (F) | paraffin oil, viscous | 2.5 |
| (G) | coco-caprylate/caprate (e.g. Cetiol LC) | 2.5 |
| (H) | Carbopol 974 P | 1.0 |
| (I) | diethylamine | 0.7 |
| (J) | sodium sulphite | 0.1 |
| (K) | water, demineralized | ad 100.0 |

(i) H is dispersed in a portion of K by means of a rotor-stator homogeniser.
(ii) A solution of B, I, J and D in C as well as the remaining K is added thereto and distributed homogeneously.
(iii) To form the fatty phase, E, G and F are melted together at 75°. A is added to the fatty phase, and then the whole fatty phase is slowly added to the previously formed gel (ii) and emulsified.

### Example 4: An emulsion-gel comprising 1% clotrimazole and 0.5% diclofenac sodium is manufactured as follows.

| Ingredients | | Amount (g/100g) |
|---|---|---|
| (A) | clotrimazole | 1.0 |
| (B) | diclofenac sodium | 0.5 |
| (C) | isopropyl myristate | 10.0 |
| (D) | Polysorbate 20 | 5.0 |
| (E) | sorbitan monolaurate | 1.0 |
| (F) | benzyl alcohol | 0.5 |
| (G) | Carbopol 974 P | 1.0 |
| (H) | sodium hydroxide | 0.1 |
| (I) | ethanol 96% (v/v) | 10.0 |
| (J) | water, demineralized | ad 100.0 |

The emulsion-gel is manufactured in a manner analogous to Example 2.

### Example 5: An emulsion-gel comprising 1% terbinafine free base and 0.1% diclofenac sodium is manufactured as follows.

| Ingredients | | Amount (g/100g) |
|---|---|---|
| (A) | terbinafine free base | 1.0 |
| (B) | diclofenac sodium | 0.1 |
| (C) | isopropanol | 20.0 |
| (D) | propylene glycol | 5.0 |
| (E) | Cetomacrogol 1000 (polyhydroxyethylene cetyl stearyl ether) | 2.0 |
| (F) | paraffin oil, viscous | 2.5 |
| (G) | Cetiol LC (coco-caprylate/caprate) | 2.5 |
| (H) | Carbopol 980 (carbomer) | 1.4 |
| (I) | ammonia (conc. aqueous solution) | 1.4 |
| (J) | sodium sulphite | 0.1 |
| (K) | water, demineralized | ad 100.0 |

The emulsion-gel is manufactured in a manner analogous to Example 3.

### Example 6: An emulsion-gel comprising 1% terbinafine free base and 0.5% indomethacin sodium is manufactured as follows.

| Ingredients | | Amount (g/100g) |
|---|---|---|
| (A) | terbinafine free base | 1.0 |
| (B) | indomethacin sodium | 0.5 |
| (C) | isopropyl myristate | 10.0 |
| (D) | Polysorbate 20 | 5.0 |
| (E) | sorbitan monolaurate | 1.0 |
| (F) | benzyl alcohol | 0.5 |
| (G) | Carbopol 974 | 1.0 |
| (H) | sodium hydroxide | 0.1 |
| (I) | ethanol | 10.0 |
| (J) | water, demineralized | ad 100.0 |

The emulsion-gel is manufactured in a manner analogous to Example 2.

### Example 7: An emulsion-gel comprising 1% terbinafine free base and 0.5% indomethacin sodium is manufactured as follows.

| Ingredients | | Amount (g/100g) |
|---|---|---|
| (A) | terbinafine free base | 1.0 |
| (B) | indomethacin sodium | 0.5 |
| (C) | isopropanol | 10.0 |
| (D) | propylene glycol | 5.0 |
| (E) | Cetomacrogol 1000 | 2.0 |
| (F) | paraffin, liquid | 2.5 |
| (G) | Cetiol LC | 2.5 |
| (H) | Carbopol 974 P | 1.4 |
| (I) | ammonia (conc. aqueous solution) | 1.4 |
| (K) | water, demineralized | ad 100.0 |

The emulsion-gel is manufactured in a manner analogous to Example 3.

## Claims

1. A pharmaceutical composition adapted to topical administration, which comprises an antifungal drug selected from the group consisting of terbinafine and topically acceptable salts thereof and a second drug selected from the group consisting of diclofenac, indomethacin and topically acceptable salts of any of said two compounds, together with at least one topically acceptable carrier.

2. A composition according to claim 1, wherein the antifungal drug is terbinafine, or a topically acceptable salt thereof, and the second drug is diclofenac, or a topically acceptable salt thereof.

3. A composition according to claim 1 or claim 2, which comprises, as antifungal drug, terbinafine or terbinafine hydrochloride.

4. A composition according to any one of claims 1-3, which comprises, as second drug, diclofenac, diclofenac sodium, diclofenac potassium, diclofenac diethylammonium or diclofenac epoiamine.

5. A composition according to any one of claims 1-4, wherein the antifungal drug is present in a weight percentage of from 0.1% up to 10% and the second drug is present in a weight percentage of from 0.05% up to 10% of the total composition.

6. A composition according to any one of claims 1-4, wherein the antifungal drug is present in a weight percentage of from 0.5% up to 2% and the second drug is present in a weight percentage of from 0.1% up to 2% of the total composition.

7. A composition according to any one of claims 1-6, which is in the form of an emulsion-gel, a gel, a foam gel, a cream, a lotion or a solution, a shampoo or a nail lacquer.

8. A composition according to any one of claims 1-6, which is in the form of an emulsion comprising
an oily phase comprising an antifungal drug as defined in said claim, and
an aqueous phase comprising water, one or more solvents selected from the group consisting of C₁-C₄-alkanols, poly-hydroxy-C₂-C₅-alkanes and poly-C₂-C₅-alkylene glycols, a water-soluble or water-miscible nonionic surfactant, wherein no anionic surfactant is present, and a second drug as defined in said claim.

9. A composition according to claim 8, wherein the Oily phase comprises an antifungal drug selected from the group consisting of terbinafine and topically acceptable salts thereof, and the aqueous phase comprises water, a C₁-C₄-alkanol, a water-soluble or water-miscible nonionic surfactant, wherein no anionic surfactant is present, and a second drug selected from the group consisting of diclofenac, indomethacin and topically acceptable salts of any of said two compounds.

10. A composition according to claim 9, wherein the second drug is diclofenac, or a topically acceptable salt thereof.

11. A composition according to any one of claims 8-10, wherein the oil forming the oily phase is isopropyl myristate or a mixture of coco-caprylate/caprate and liquid paraffin.

12. A composition according to any one of claims 8-11, wherein the weight ratio of the antifungal drug and the oil forming the oily phase is of from 1:3 up to 1:40.

13. A composition according to any one of claims 8-11, wherein the weight ratio of the antifungal drug and the second drug is of from 1:0.05 up to 1:5.

14. Use of an antifungal drug selected from the group consisting of terbinafine and topically acceptable salts thereof, and a second drug selected from the group consisting of diclofenac, indomethacin, and topical acceptable salts of any of said two compounds, for the manufacture of a pharmaceutical composition adapted to topical administration for the prevention or treatment of fungal infections.

15. Use according to claim 14, where the pharmaceutical composition manufactured is useful in fighting dermatophytes.

## Patentansprüche

1. Pharmazeutische Zusammensetzung, angepasst zur topischen Verabreichung, die einen antifungalen Arzneistoff, ausgewählt aus der Gruppe, bestehend aus Terbinafin und topisch verträglichen Salzen davon, und einen zweiten Arzneistoff, ausgewählt aus der Gruppe, bestehend aus Diclofenac, Indomethacin und topisch verträglichen Salzen von beliebigen der zwei Verbindungen, zusammen mit mindestens einem topisch verträglichen Träger umfasst.

2. Zusammensetzung nach Anspruch 1, wobei der antifungale Arzneistoff Terbinafin oder ein topisch verträgliches Salz davon ist und der zweite Arzneistoff Diclofenac oder ein topisch verträgliches Salz davon ist.

3. Zusammensetzung nach Anspruch 1 oder Anspruch 2, die als einen antifungalen Arzneistoff Terbinafin oder Terbinafinhydrochlorid umfasst.

4. Zusammensetzung nach einem der Ansprüche 1-3, die als zweiten Arzneistoff Diclofenac, Diclofenacnatrium, Diclofenackalium, Diclofenacdiethylammonium oder Diclofenacepolamin umfasst.

5. Zusammensetzung nach einem der Ansprüche 1-4, wobei der antifungale Arzneistoff in einem Gewichtsprozentsatz von 0,1% bis zu 10% vorliegt und der zweite Arzneistoff in einem Gewichtsprozentsatz von 0,05% bis zu 10% der Gesamtzusammensetzung vorliegt.

6. Zusammensetzung nach einem der Ansprüche 1-4, wobei der antifungale Arzneistoff in einem Gewichtsprozentsatz von 0,5% bis zu 2% vorliegt und der zweite Arzneistoff in einem Gewichtsprozentsatz von 0,1% bis zu 2% der Gesamtzusammensetzung vorliegt.

7. Zusammensetzung nach einem der Ansprüche 1-6, die in Form eines Emulsionsgels, eines Gels, eines Schaumgels, einer Creme, einer Lotion oder einer Lösung, eines Shampoos oder eines Nagellacks vorliegt.

8. Zusammensetzung nach einem der Ansprüche 1-6, die in Form einer Emulsion vorliegt, umfassend
eine ölige Phase, umfassend einen antifungalen Arzneistoff wie in dem Anspruch definiert und
eine wässrige Phase, umfassend Wasser, ein oder mehrere Lösungsmittel, ausgewählt aus der Gruppe, bestehend aus C₁-C₄-Alkanolen, Poly-hydroxy-C₂-C₅-alkanen und Poly-C₂-C₅-alkylenglycolen, einem in Wasser löslichen oder mit Wasser mischbaren nichtionischen Tensid, wobei kein anionisches Tensid vorliegt, und einen zweiten wie in dem Anspruch definierten Arzneistoff.

9. Zusammensetzung nach Anspruch 8, wobei die ölige Phase einen antifungalen Arzneistoff, ausgewählt aus der Gruppe, bestehend aus Terbinafin und topisch verträglichen Salzen davon, umfasst und die wässrige Phase Wasser, ein C₁-C₄-Alkanol, ein in Wasser lösliches oder mit Wasser mischbares nichtionisches Tensid, wobei kein anionisches Tensid vorliegt, und einen zweiten Arzneistoff, ausgewählt aus der Gruppe, bestehend aus Diclofenac, Indomethacin und topisch verträglichen Salzen von beliebigen der zwei Verbindungen, umfasst.

10. Zusammensetzung nach Anspruch 9, wobei der zweite Arzneistoff Diclofenac oder ein topisch verträgliches Salz davon ist.

11. Zusammensetzung nach einem der Ansprüche 8-10, wobei das die ölige Phase bildende Öl Myristinsäureisopropylester oder ein Gemisch von Coco-caprylat/Caprat und flüssigem Paraffin ist.

12. Zusammensetzung nach einem der Ansprüche 8-11, wobei das Gewichtsverhältnis des antifungalen Arzneistoffs und des die ölige Phase bildenden Öls von 1:3 bis zu 1:40 ist.

13. Zusammensetzung nach einem der Ansprüche 8-11, wobei das Gewichtsverhältnis des antifungalen Arzneistoffs und des zweiten Arzneistoffs 1:0,05 bis zu 1:5 ist.

14. Verwendung eines antifunglen Arzneistoffs, ausgewählt aus der Gruppe, bestehend aus Terbinafin und topisch verträglichen Salzen davon und eines zweiten Arzneistoffs, ausgewählt aus der Gruppe, bestehend aus Diclofenac, Indomethacin und topisch verträglichen Salzen von beliebigen der zwei Verbindungen, zur Herstellung einer pharmazeutischen Zusammensetzung, angepasst zur topischen Verabreichung für die Verhinderung oder Behandlung von Pilzinfektionen.

15. Verwendung nach Anspruch 14, wobei die hergestellte pharmazeutische Zusammensetzung zum Bekämpfen von Dermatophyten verwendbar ist.

## Revendications

1. Composition pharmaceutique pour administration topique comprenant un produit antifongique sélectionné à partir du groupe composé de la terbinafine et de ses sels acceptables d'un point de vue topique et un second produit sélectionné à partir du groupe composé du diclofénac, de l'indométhacine ainsi que de sels acceptables d'un point de vue topique de chacun de ces deux composés, de pair avec au moins un véhicule acceptable d'un point de vue topique.

2. Composition selon la revendication 1, **caractérisée en ce que** le produit antifongique est la terbinafine ou un de ses sels acceptables d'un point de vue topique et **en ce que** le second produit est le diclofénac ou un de ses sels acceptables d'un point de vue topique.

3. Composition selon la revendication 1 ou 2 comprenant, en tant que produit antifongique, la terbinafine ou le chlorhydrate de terbinafine.

4. Composition selon l'une des revendications 1 à 3 comprenant, en tant que second produit, le diclofénac, le diclofénac de sodium, le diclofénac de potassium, le diclofénac de diéthylammonium ou le diclofénac d'épolamine.

5. Composition selon l'une des revendications 1 à 4, **caractérisé en ce que** le produit antifongique est présent selon un pourcentage de 0,1% à 10% en poids et **en ce que** le second produit est présent selon un pourcentage de 0,05% à 10% en poids de la composition totale.

6. Composition selon l'une des revendications 1 à 4, **caractérisé en ce que** le produit antifongique est présent selon un pourcentage de 0,5% à 2% en poids et **en ce que** le second produit est présent selon un pourcentage de 0,1% à 2% en poids de la composition totale.

7. Composition selon l'une des revendications 1 à 6 sous forme d'une émulsion-gel, d'un gel, d'un gel moussant, d'une crème, d'une lotion ou d'une solution, d'un shampoing ou d'un vernis à ongles.

8. Composition selon l'une des revendications 1 à 6 sous forme d'une émulsion comprenant
- une phase huileuse comprenant un produit antifongique tel que défini dans lesdites revendications, et
- une phase aqueuse comprenant de l'eau, un ou plusieurs solvants sélectionnés à partir du groupe composé de C₁-C₄-alkanols, de poly-hydroxy-C₂-C₅-alkanes et de poly-C₂-C₅-alkylène glycols, d'un tensioactif non ionique hydrosoluble ou miscible à l'eau dans lequel aucun tensioactif anionique n'est présent et d'un second produit tel que défini dans lesdites revendications.

9. Composition selon la revendication 8, **caractérisée en ce que** la phase huileuse comprend un produit antifongique sélectionné à partir du groupe composé de terbinafine et de ses sels acceptables d'un point de vue topique et **en ce que** la phase aqueuse comprend un C₁-C₄-alkanol, un tensioactif non ionique hydrosoluble ou miscible à l'eau dans lequel aucun tensioactif anionique n'est présent et, un second produit sélectionné à partir du groupe composé du diclofénac, de l'indométhacine ainsi que des sels acceptables d'un point de vue topique de chacun de ces deux composés.

10. Composition selon la revendication 9, **caractérisée en ce que** le second produit est le diclofénac ou un de ses sels acceptables d'un point de vue topique.

11. Composition selon l'une des revendications 8 à 10, **caractérisée en ce que** l'huile formant la phase huileuse est le myristate d'isopropyle ou un mélange de coco-caprylate/caprate et de paraffine liquide.

12. Composition selon l'une des revendications 8 à 11, **caractérisée en ce que** le rapport pondéral entre le produit antifongique et l'huile formant la phase huileuse est de 1:3 à 1:40.

13. Composition selon l'une des revendications 8 à 11, **caractérisée en ce que** le rapport pondéral entre le produit antifongique et le second produit va de 1:0,05 à 1:5.

14. Utilisation d'un produit antifongique sélectionné à partir du groupe composé de terbinafine et de ses sels acceptables d'un point de vue topique ainsi que d'un second produit sélectionné à partir du groupe composé du diclofénac, de l'indométhacine ainsi que des sels acceptables d'un point de vue topique de chacun de ces deux composés, dans la fabrication d'une composition pharmaceutique pouvant être administrée de façon topique pour le traitement ou la prévention d'infections fongiques.

15. Utilisation selon la revendication 14, **caractérisée en ce que** la composition pharmaceutique ainsi fabriquée est utile pour combattre les dermatophytes.
